# EUROPEAN PATENT SPECIFICATION

(11) **EP 2 724 747 B1**
(45) Date of publication and mention of the grant of the patent: **23.09.2020**
(21) Application number: 13190551.5
(22) Date of filing: 28.10.2013
(51) Int. Cl.: A61N 5/06

(54) **Medical device intended for light therapy for patients with chronic skin disease**
Lichttherapiegerät
Appareil pour phototherapie

(30) Priority: 26.10.2012 NL 2009708
(43) Date of publication of application: 30.04.2014
(73) Proprietor: Sunshower Medical B.V., 1099 DK Duivendrecht (NL)
(72) Inventor: van Vijfeijken, Iwan Lukas, 5521 CK Eersel (NL); Wegdam, Pieter Merijn, 1078 BC Amsterdam (NL)
(74) Representative: Verhees, Godefridus Josephus Maria

(56) References cited:
- EP-A1- 0 255 426
- WO-A1-85/00527
- WO-A1-2005/034165
- WO-A1-2009/004412
- WO-A2-03/039671
- DE-A1- 2 803 446
- DE-U1- 8 508 206
- US-A- 4 298 005

## Description

### Field of the invention

The invention relates to a medical appliance intended for light therapy for patients who have a chronic dermatological disorder, comprising a UV lamp and a filter unit that filters the light emanating from the UV lamp, which filter unit comprises two filter plates which are placed parallel to one another and are interspaced. UV light is safe for the skin, provided that the exposure time is not too long and frequent and the light has a suitable, relatively low, intensity.

### State of the art

An appliance comprising a UV lamp and a filter unit that filters the light emanating from the UV lamp and which filter unit comprises two filter plates is known from EP0255426A.

### Summary of the invention

It is an object of the invention to improve the known appliance. For this purpose the medical appliance according to the invention is characterized in that the UV lamp and filter unit are such that in the wavelength range between 280 and 320 nanometers light is present having a maximum intensity of 0.35 W/m² at 40 cm distance from the UV lamp, and in that in the wavelength range from 280 to 320 nm each filter plate has a transmission factor increasing from about zero at 280 nm to about 0.35 at 320 nm. It has turned out that light in this wavelength range has a positive effect on people who suffer from chronic dermatological disorders, provided that these people are not exposed to such light having a high intensity. The medical appliance according to the invention is a safe appliance for light therapy because of the use of the filter unit, since the light intensity is relatively low and is suitable for use for a number of times per week for relatively brief periods of time (exposure for a couple of minutes at a time).

The UV lamp and filter unit according to the invention are such that light with a wavelength of less than 280 nanometers is substantially completely absorbed. Light having a wavelength of less than 280 nanometers may be injurious to a person's health. By arranging the filter unit as two interspacedly disposed plates there is a natural cooling by the warm current of air rises up between the plates.

### Brief description of the drawings

The invention will now be described in more detail based on an example of embodiment of the medical appliance according to the invention while reference is made to the appended drawing figures, in which:
Fig. 1 gives a diagrammatic representation of the medical appliance according to the invention comprising a UV lamp and two filter plates;
Fig. 2 gives a graphical representation of the light intensity of the lamp in W/m² plotted against the wavelength; and
Fig. 3 shows the transmission percentage of a filter plate T in % plotted against the wavelength.

### Detailed description of the drawings

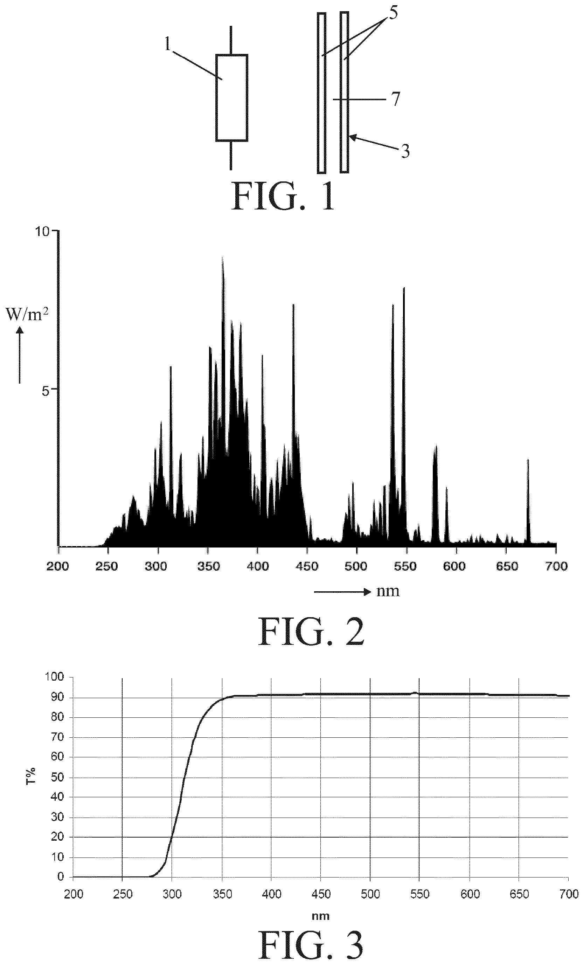

Fig. 1 gives a diagrammatic representation of the medical appliance according to the invention. The appliance comprises a UV lamp 1 as well as a filter unit 3. The filter unit is formed by two parallel interspacedly disposed filter plates 5 having between them a space 7 which is connected to the environment. With the aid of the medical appliance it is possible to attend to patients who have a chronic dermatological disorder. The UV lamp and filter unit are such that in the wavelength range between 280 and 320 nanometers light is present having a maximum intensity of 0.35 W/m² at a distance of 40 cm from the UV lamp. It has turned out that light in this wavelength range has a positive effect on persons who suffer from a sun allergy or psoriasis. The filter unit prevents these people from being exposed to such light that has a high intensity.

For the UV lamp has been chosen in favour of the Cosmedico Ariana N 400 R7s metal halogenide lamp or similar and for the filter plate has been chosen in favour of the DiamantClass clear glass filter manufactured by Messrs. Hecker Glastechnik GmbH & Co KG, Schleefstrasse 5, 44287 Dortmund, Germany. The material of this filter is soda lime glass.

For illustrative purposes Fig. 2 shows in a graph the light intensity of the lamp in W/m² as a function of the wavelength and in Fig. 3 is plotted the transmission percentage of a single filter plate T against the wavelength. In the wavelength range from 280 to 320 nm each filter plate has a maximum transmission factor of 0.35. The two filter plates combined have a maximum transmission factor of 0.105. Light having a wavelength of less than 280 nanometers is substantially completely absorbed by the filter plates to prevent damaging effects.

By arranging the filter unit 3 as two interspacedly disposed plates 5, a natural cooling takes place which causes of the warm current of air to rise up between the plates as a result of natural convection.

Albeit the invention has been described in the foregoing with reference to the drawings, it should be pointed out that the invention is not by any manner or means restricted to the embodiments shown in the drawings. The invention also extends over any embodiment deviating from the embodiments shown in the drawings within the scope defined by the claims.

## Claims

1. Medical appliance intended for light therapy for patients who have a chronic dermatological disorder, comprising a UV lamp and a filter unit that filters the light emanating from the UV lamp such that light having a wavelength of less than 280 nm is completely absorbed, wherein the filter unit comprises two filter plates which are placed parallel to one another and are interspaced, **characterized in that** the UV lamp and filter unit are such that in the wavelength range between 280 and 320 nanometers light is present having a maximum intensity of 0.35 W/m² at 40 cm distance from the UV lamp, and **in that** in the wavelength range from 280 to 320 nm each filter plate has a transmission factor increasing from zero at 280 nm to 0.35 at 320 nm.

## Patentansprüche

1. Medizinisches Gerät zur Lichttherapie für Patienten mit chronisch dermatologischer Störung, das einer UV-Lampe und einer Filtereinheit umfasst, wobei der Filtereinheit das von der UV-Lampe ausgehende Licht so filtert, dass Licht mit einer Wellenlänge von weniger als 280 nm vollständig absorbiert wird, wobei der Filtereinheit zwei Filterplatten umfasst, die parallel zueinander angeordnet und voneinander beabstandet sind, **dadurch gekennzeichnet, dass** die UV-Lampe und die Filtereinheit so beschaffen sind, dass im Wellenlängenbereich zwischen 280 und 320 Nanometer Licht mit einer maximalen Intensität von 0,35 W/m² auf 40 cm Abstand von der UV-Lampe vorhanden ist, und im Wellenlängenbereich von 280 bis 320 nm jede Filterplatte einen Transmissionsfaktor hat, der von Null bei 280 nm auf 0,35 bei 320 nm ansteigt.

## Revendications

1. Appareil médical destiné à la luminothérapie pour les patients souffrant d'un trouble dermatologique chronique, comprenant une lampe UV et une unité de filtre qui filtre la lumière émanant de la lampe UV de telle sorte que la lumière d'une longueur d'onde inférieure à 280 nm soit complètement absorbée, dans lequel le filtre l'unité comprend deux plaques filtrantes placées parallèlement l'une à l'autre et espacées, **caractérisée en ce que** la lampe UV et l'unité de filtre sont telles que dans la gamme de longueurs d'onde entre 280 et 320 nanomètres une lumière est présente avec une intensité maximale de 0,35 W/m2 à 40 cm de la lampe UV, et **en ce que** dans la gamme de longueurs d'onde de 280 à 320 nm, chaque plaque filtrante a un facteur de transmission croissant de zéro à 280 nm à 0,35 à 320 nm.
